# EUROPEAN PATENT APPLICATION

(11) **EP 2 153 837 A1**
(43) Date of publication of application: **17.02.2010**
(21) Application number: 08290773.4
(22) Date of filing: 14.08.2008
(51) Int. Cl.: A61K 35/74, A61P 11/00

(54) **Compositions comprising Lactobacillus casei for improving resistance to common infectious diseases**

(71) Applicant: Compagnie Gervais Danone, 75009 Paris (FR)
(72) Inventor: Guillemard, Eric, 75015 Paaris (FR); Flavigny, Laure, 75015 Paris (FR); Tanguy, Jerome, 94230 Cachan (FR)
(74) Representative: Vialle-Presles, Marie José

(57) **Abstract**

The use of a *Lactobacillus casei* strain for improving the resistance of a tobacco user against a common infectious disease, especially against a respiratory common infectious disease, and for improving the cellular immune response of an individual in case of respiratory common infectious disease.

## Description

The present invention relates to compositions for improving resistance to common infectious diseases.

Common infectious diseases (CID), defined as lower and upper respiratory tract infections and gastroenteritis, are endemic in the general population, causing considerable amounts of discomfort.

The different types of common infectious diseases in each category and their main associated symptoms used for diagnosis are summarized in Table 1 below.

**Table 1**

| Common infectious diseases | Symptoms |
|---|---|
| Upper respiratory tract infections (URTI) | |
| Rhinopharyngitis (cold, acute coryza) | Rhinorhea, sneezing, nasal congestion, headache, asthenia, sore/stiff muscles, fever (rare) |
| Sore throat | Fever (except viral infection), burning throat pain, red, swollen, bubbling tonsils, adenopathy with neck pain. |
| Acute sinusitis | Headaches, sinus pain, stuffy nose, purulent rhinorrhea, sore throat, cough, fever. |
| Acute otitis | Ear pain, sensation that ear is full, hearing impairment, discharge. |
| Lower respiratory tract infections (LRTI) | |
| Acute bronchitis | Moderate fever, cough, mucous or purulent expectoration. |
| Pneumopathy | Fever generally high, shivers, cough, mucous or purulent expectoration, chest pain, dyspnea. |
| Flu and flu-like illness | Intense shivers, high fever, headaches, sore/stiff muscles, arthralgia, asthenia, anorexia, dry, painful cough, red pharynx. |
| Gastro-intestinal tract infection (GiTi) | |
| - Gastroenteritidis | Fever, headaches, sore/stiff muscles, abdominal pain, diarrhoea, vomiting. |

In 2002, the common cold was responsible for millions of days missed from work and school and accounted for 27 million physician visits in the United States (Greenberg 2002), while Feeney showed that respiratory disorders and gastroenteritis accounted for 50 to 60 % of all spells of absence in London (Feeney A *et al*. 1998). Furthermore, upper respiratory tract infections are the leading reason for inappropriate antibiotic use in both children and adults, leading to increased spread of antibiotic resistance. At last, for some infections such as common colds induced by rhinoviruses, no antiviral drugs have been approved (Greenberg 2002). So, common infections are of great interest in terms of public health with socioeconomic consequences.

Stress is one of the factors that can act in increasing the risk of being infected, due to its action on the immune system. Indeed, studies have shown that psychological stress can down-regulate immune responses by causing the dysregulation of different signals. Some "pathways" by which the immune system is modulated upon psychological stress have been demonstrated. For example, there is direct innervation of primary and secondary lymphoid tissues by the autonomic nervous system. These "pathways" operate by producing biological mediators that interact with and affect cellular components of the immune system (Yang EV & Glaser R. 2000). Thus, Yang and Glaser demonstrated that psychological stressors have the ability to modulate the cellular immune response that can result in the increase of an individual's susceptibility to infectious pathogens (Yang EV & Glaser R. 2000).

Several situations and activities can generate stress, among which work activity is of particular interest. Indeed, the impact of stressful conditions of work on infections has been demonstrated by studies conducted especially in shift-workers. Epidemiological studies demonstrated that the occurrence of common cold, flu-like illness and gastroenteritis is different among employees in different work schedules. Shift work was significantly associated with higher incidence for all three common infections (Mohren DC *et al*. 2002). A few studies have reported depressed immune function in relation to shift-work, which may explain an increased susceptibility to infections (Curti R *et al*. 1982; Kobayashi F *et al*. 1997).

Among the products potentially active against common infections, probiotics have shown interesting effects especially in adults workers. For example, *Lactobacillus reuteri* ATCC55730 have been shown to decrease the short-term sick-leaves caused by respiratory or gastrointestinal infections, and thus improved work-place healthiness (Tubelius *et al*. 2005). In other studies, anti-infectious effect of probiotics have been correlated with modulation of immune parameters. In adults, a mix of probiotics (*i*.*e*. *Lactobacillus gasseri* PA 16/8, *Bifidobacterium longum* SP 07/3 and *Bifidobacterium bifidum* MF 20/5) was reported to decrease the severity and the duration of common cold infections and the number of days of associated fever. In this study, improvement of disease outcome was associated to a significant increase of cytotoxic and suppressor CD8 T cells count in the group having consumed probiotics (De Vrese *et al*. 2005).

DANONE has developed a probiotic dairy drink, ACTIMEL^{®}, containing *Lactobacillus casei* DN-114 001 as well as characteristic yoghurt cultures (*Lactobacillus bulgaricus* and *Streptococcus thermophilus*).

ACTIMEL^{®} consumption was reported to have a beneficial effect on the outcome of gastro-intestinal diseases. In children attending day care centres, ACTIMEL^{®} supplementation reduced the incidence of acute diarrhoea as compared to a yogurt supplemented control group (Pedone *et al*. 2000). With the same regimen, a significant decrease of the duration of acute diarrhoea was also observed in children (Pedone *et al*. 1999, Agarwal *et al*. 2001). A study was also conducted in a population of elderly people. The effect of a 3-week ACTIMEL^{®} consumption on the incidence, duration and associated fever of both gastro-intestinal and respiratory winter infections was investigated. In this study, ACTIMEL^{®} consumption reduced the duration of disease in comparison with control group (Turchet *et al*. 2003).

ACTIMEL^{®} was also shown to modulate some immune cells count or activities in several populations including stressed subjects. Consumption of ACTIMEL increases or inhibits the decrease of some immune parameters (total lymphocytes, CD56⁺ cells, *i*.*e*., Natural Killer + T cytotoxic) in subjects 18-23 years old submitted to academic examination stress (Marcos *et al*. 2004). In the same way, in a model of highly trained athletes, in whom physical activity has acute and chronic effects on the immune system, the decrease in the number of NK cells was significantly lower after consumption of ACTIMEL^{®} than after consumption of milk (Pujol *et al*. 2000). With an 8-weeks period of product consumption, other studies carried out in a middle-aged adult population (51-58 years) showed that ACTIMEL^{®} increases both NK cells cytotoxic activity (Parra *et al*. 2004a; Parra *et al*. 2004b) and oxidative burst activity of monocytes (Parra *et al*. 2004b). Therefore those results showed that ACTIMEL^{®} can regulate cellular immunity especially the innate one, both by increasing subset size or specific activities of immune cells.

The inventors have now investigated the effect of ACTIMEL^{®} on adults resistance against common infections, especially in immunocompetent adults younger than 65 working in stressful conditions. As shown in the experimental part below, they demonstrated that the effect of ACTIMEL^{®} consumption is greater in tobacco users than in non smokers. Moreover, the inventors have shown that in the general population, regular uptake of ACTIMEL^{®} leads to a stronger immune response in case of CID, since both NK cells and neutrophilic leukocytes act.

The present invention hence pertains to the use of a *Lactobacillus casei* strain, for the preparation of a composition for improving the resistance of a tobacco user, in particular a smoker, against a common infectious disease, especially against a respiratory common infectious disease. It also pertains to the use of a *Lactobacillus casei* strain, for the preparation of a composition for improving the cellular immune response, in particular the NK cells response and/or the neutrophilic leukocytes of an individual in case of a respiratory common infectious disease, such as rhinopharyngitis or sore throat.

Preferably, said composition is indented to be administered to a subject who is younger than 65 years old.

According to a preferred embodiment of the invention, said *Lactobacillus casei* strain is a *Lactobacillus casei* ssp. *paracasei* strain, preferably the strain deposited at the CNCM under the reference I-1518, which is described for instance in EP0794707 or EP1283714.

Advantageously, said composition comprises at least 1 × 10⁵ c.f.u. per millilitre of said *Lactobacillus casei* strain, more preferably least 1 × 10⁷ c.f.u. per millilitre of said *Lactobacillus casei* strain. Said strain can be associated with one or more other lactic acid bacteria selected from the genera *Lactobacillus, Lactococcus, Streptococcus and Bifidobacterium*, the preferred lactic acid bacteria being those chosen from a group comprising *Lactobacillus helveticus, Lactobacillus delbrueckii* subspecies *bulgaricus, Lactobacillus rhamnosus, Lactobacillus acidophilus, Lactococcus lactis, Streptococcus thermophilus, Bifidobacterium longum* and/or *Bifidobacterium breve*. In a particularly preferred embodiment, said *Lactobacillus casei* strain is associated with *Lactobacillus bulgaricus* and/or *Streptococcus thermophilus* bacteria.

Said composition can be in particular in the form of an aliment or of a food complement. It is preferably a fermented milk composition.

The present invention will be further illustrated by the following additional description, which refers to examples illustrating the properties of a composition comprising *Lactobacillus casei* for improving the resistance against common infectious diseases.

### EXAMPLE 1 : MATERIALS AND METHODS

### 1.1 : Population studied

### Demography :

The characteristics of the population of the study are summarized in tables 2 and 3 below.

**Table 2 : characteristics of the population studied**

| | ACTIMEL^{®} N=500 | Control | All | p-value |
|---|---|---|---|---|
| | | N=500 | N=1000 | |
| Age (mean ± SD) | 31.8 ± 8.9 | 32.5 ± 8.9 | 32.1 ± 8.9 | (ANOVA) |
| Q1-Q3 (50% of population) | 25-37 | 26-38 | 25-38 | 0.278 |

| Sex | | | | |
|---|---|---|---|---|
| Women | 57% | 56% | 56% | (X²) |
| Men | 43% | 44% | 44% | 0.702 |
| BMI (mean ± SD) | 24.0 ± 2.8 | 24.2 ± 2.9 | 24.1 ± 2.9 | (ANOVA) |
| Q1-Q3 (50% of population) | 22-26 | 22-26 | 22-26 | 0.235 |

| Type of activity | | | | |
|---|---|---|---|---|
| Worker in factory | 4.25% | 4.6% | 4.4% | |
| Nurse | 47% | 45% | 46% | (X²) |
| Fire fighter | 5.8% | 6.0% | 5.9% | 0.993 |
| Police officer | 16% | 16% | 16% | |
| Other | 27% | 28% | 28% | |

| Baseline characteristics | | | | |
|---|---|---|---|---|
| Type of shift-work | | | | |
| 2-shift work | 17% | 18% | 18% | (X²) |
| 3-shift work | 83% | 82% | 83% | 0.803 |

| Smoking habit | | | | |
|---|---|---|---|---|
| Non-smoker | 42% | 41% | 42% | |
| Smoker < 10 cig./d. | 21% | 18% | 19% | (X²) |
| Smoker > 10 cig./d. | 18% | 18% | 18% | 0.190 |
| Former smoker | 19% | 22% | 21% | |
| Other | <1% | 1.4% | <1% | |

| Vaccination against influenza | | | | |
|---|---|---|---|---|
| Yes | 7.0% | 5.4% | 6.2% | (X²) |
| No | 93% | 95% | 94% | 0.294 |

**Table 3 : Study inclusion and withdrawal**

| Number of subjects | | ACTIMEL^{®} | Control | All |
|---|---|---|---|---|
| Screened (Screened population) | | | | N=1523 |
| Enrolled (Total population) | Yes | | | 1000 (66%) |
| | No | | | 523 (34%) |
| Reason for non inclusion | Non-eligible subject | | | 158 (10%) |
| | Enrolment closed | | | 252 (17%) |
| | Subject's decision | | | 25(2%) |
| | Non compliant | | | 10 (0.6%) |
| | CID (at V1 or V2) | | | 72 (5%) |
| | Other | | | 6 (0.4%) |
| Randomised | ITT population | N=500 | N=500 | N=1000 |
| | PP population | N=443 | N=457 | N= 900 |

### Exclusions and premature study withdrawals

A total of 38 subjects in the ITT population (4%) withdrew before the end of the study, while all subjects included in the PP population completed the study (by definition).

**Table 4: Premature study withdrawals**

| | ACTIMEL^{®} | Control | All |
|---|---|---|---|
| Number of subjects | (N=500) | (N=500) | (N=1000) |

| Completion Status | | | |
|---|---|---|---|
| completed | 478 (96%) | 484 (97%) | 962 (96%) |
| discontinued | 22 (4.4%) | 16 (3.2%) | 38 (3.8%) |

### 1.2. Identity of investigational products

### Products characteristics

The products given during this study belong to the family of fresh dairy products. The present study was conducted by using ACTIMEL^{®} as active product.

Table 5 provides the composition of the active and the control products.

To preserve double blind methodology, the active and the control product appearance, packaging, and taste were identical.

The active product (ACTIMEL^{®}) is a sweetened flavoured fermented dairy drink.

The control product is a sweetened flavoured non-fermented acidified dairy drink.

**Table 5: Product composition. cfu: colony forming unit.**

| Product | Lipids | Glucids | Proteins | *Lactobacillus casei* | Yogurt culture |
|---|---|---|---|---|---|
| ACTIMEL^{®} | 1.5% | 12% | 2.8% | minimum 1 × 10⁸ cfu/mL | minimum 5 × 10⁷ cfu/mL |
| Control | 1.5% | 12 % | 2.8% | -- | -- |

### 1.3. Study plan

### Description of the study plan

The study was performed in 1 centre, randomised with a stratification by type of activities, double blinded, controlled in 2 parallel groups: "ACTIMEL^{®} product" vs. "Control product".

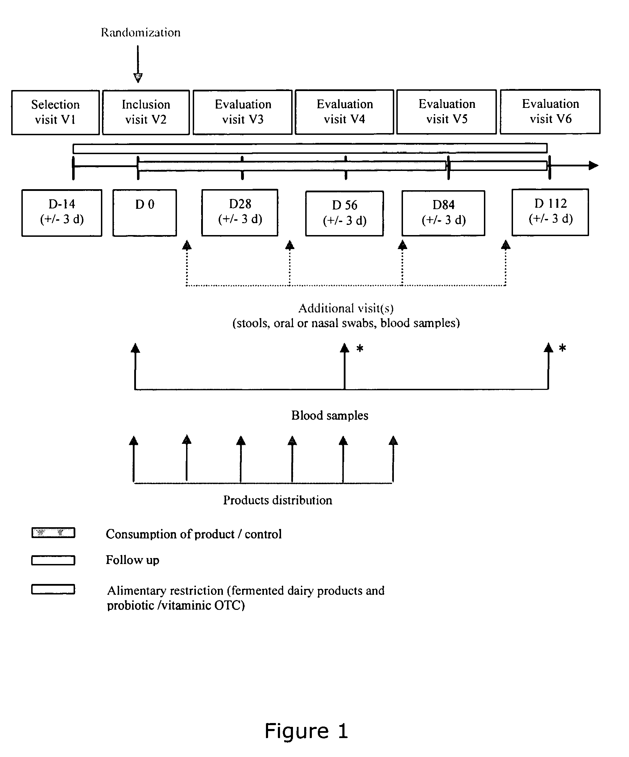

Figure 1 displays the timing of the study procedures (*: Depending on the participation to the sub-group on "Blood biological parameters").

After the screening phase (V1-V2), the experimental phase is divided in 2 parts: a phase of consumption of products (V2-V5: 12 weeks) and a follow-up phase (V5-V6: 4 weeks) without consumption of product.

For each subject, the total duration of the study was 4.5 months (2 weeks of alimentary restriction, 12 weeks of product consumption and 4 weeks of follow-up).

### Evaluation criteria

The primary evaluation criterion of the study was the number of all CID reported by subject during the 12-weeks product consumption period. In this study, CID were defined as 3 main categories of infections:
- Upper respiratory tract infections (URTI): Rhinopharyngitis, Sore throat, Sinusitis, Otitis
- Lower respiratory tract infections (LRTI): Bronchitis, Pneumopathy, *etc*.
- Gastro-intestinal tract infections (GITI).

*Primary evaluation variables*

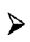 **Primary** :

Comparison between groups of the number of all CIDs reported during the 12 weeks of study product consumption.

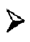 **Secondary :**

- Comparison between groups of the number of all CIDs reported during the 4-week follow-up and during the whole study phase [consumption phase + follow up]
- Comparison between groups of the number of URTI or LRTI or GITI or each type of CID during the 12 weeks of study product consumption, during the 4-weeks follow up and during the whole study phase [consumption phase + follow up]
- Comparison between groups of the number of subjects having at least 1 CID (any type), or 1 URTI or LRTI or GITI, or one type CID (*e*.*g*.: rhinopharyngitis or sore-thoat) during the 12 weeks of study product consumption, during the 4-weeks follow up and during the whole study phase [consumption phase + follow up].
- Comparison between groups of evolution, in each group, of the Hemogram at some planned visits and in case of CID (any type), or URTI or LRTI or GITI, or each type of CID during the 12 weeks of study product consumption, during the 4 weeks follow up and during the whole study phase [consumption phase + follow up].
- Comparison between groups of the number of days of fever during all CID (any type), or URTI or LRTI or GITI, or each type of CID, during the 12 weeks of study product consumption, during the 4 weeks follow up and during the whole study phase [consumption phase + follow up].
- Comparison between groups of evolution in each group of the immune system blood parameters, at some planned visits (for part of subjects) and at additional visits in case of CID (any type), or URTI or LRTI or GITI, or each type of CID, during the 12 weeks of study product consumption, during the 4 weeks follow up and during the whole study phase [consumption phase + follow up].

### 1.4. Statistical methods

### Statistical and analytical plans

The statistical analysis plan was drawn up before the Blind Review.

The statistical analysis incorporated the recommendations of the European Medicines Evaluation Agency (ICH Topic E9 - step 4 - 5 February 1998).

### Determination of sample size

The sample size determination was based on the primary outcome: the cumulated number of CID occurring within the three months of product consumption. Multiple events of different infectious diseases occurring jointly (*e*.*g*., an upper respiratory tract infection and a gastroenteritis) have been counted separately and added even if they occurred in the same infectious episode.

The exact rate of common infectious diseases episodes among shift workers within a given time period could not be easily anticipated because it may vary highly according to season conditions. However, it was assumed that an average number of 1.5 events could be observed in the controlled group. A 15% relative decrease was expected in the ACTIMEL^{®} group (*i*.*e*., the expected average number of events was 1.275 in the ACTIMEL^{®} randomized group). The distribution of the cumulated number of infectious events was assumed to be an overdispersed Poisson distribution; some overdispersion was expected since the occurrences of events within a subject are not independent.

With an expected rate of 1.5 events over the three months period in the controlled group and using a Poisson regression with a two-sided test at the 5% α level assuming moderate overdispersion (scale parameter of 1.1), around 450 subjects in each arm remaining in the study for three months were expected to be needed to detect a 15% reduction in rate with at least 80% power. A 5% drop-out rate of subjects was assumed; therefore around 500 subjects in each arm needed to be included. Therefore a total of 1000 subjects were randomised.

### EXAMPLE 2 : CLINICAL RESULTS

### 2.1: Number of all CID in the smokers sub-population

As shown in Table 6, a statistically significant product effect in favour of the ACTIMEL^{®} group on the number of all CID during the study product consumption phase in the smokers sub-population (mean= 0.6 for ACTIMEL^{®} vs. 0.8 for Control; p= 0.033 for ITT population), whereas this effect was not observed in the non-smokers population (p=0,687 for ITT population) (Table 7).

**Table 6: Intent-to-Treat Population - smokers (Phase = Consumption Phase - 12 weeks of study product consumption).**

| | | ACTIMEL^{®} | Control | All |
|---|---|---|---|---|
| Number of subjects | | (N=194) | (N=186) | (N=380) |
| Number of CID | 0 | 120 (62%) | 91 (49%) | 211 (56%) |
| | 1 | 39 (20%) | 59 (32%) | 98 (26%) |
| | 2 | 23 (12%) | 22 (12%) | 45 (12%) |
| | 3 | 8(4.1%) | 8 (4.3%) | 16 (4.2%) |
| | 4 | 3 (1.5%) | 3 (1.6%) | 6 (1.6%) |
| | 5 | 1 (< 1%) | 2 (1.1%) | 3 (< 1%) |
| | 6 | 0 (0.0%) | 1 (< 1%) | 1 (< 1%) |
| | | | | |

| | N | 194 | 186 | 380 |
|---|---|---|---|---|
| | Mean | 0.649 | 0.833 | 0.739 |
| | SD | 1.003 | 1.100 | 1.054 |
| | SEM | 0.072 | 0.081 | 0.054 |

**Table 7 : Intent-to-Treat Population - non-smokers (Phase = Consumption Phase - 12 weeks of study product consumption).**

| | | ACTIMEL^{®} | Control | All |
|---|---|---|---|---|
| Number of subjects | | (N=306) | (N=314) | (N=620) |
| Number of CID | 0 | 167 (55%) | 153 (49%) | 320 (52%) |
| | | | | |
| | 1 | 73 (24%) | 105 (33%) | 178 (29%) |
| | 2 | 40 (13%) | 35 (11%) | 75 (12%) |
| | 3 | 16 (5.2%) | 15 (4.8%) | 31 (5.0%) |
| | 4 | 7 (2.3%) | 5 (1.6%) | 12 (1.9%) |
| | 5 | 1 (< 1%) | 0 (0.0%) | 1 (< 1%) |
| | 6 | 2 (< 1%) | 1 (< 1%) | 1 (< 1%) |
| | | | | |

| | N | 306 | 186 | 380 |
|---|---|---|---|---|
| | Mean | 0.804 | 0.783 | 0.794 |
| | SD | 1.128 | 0.984 | 1.057 |
| | SEM | 0.064 | 0.056 | 0.042 |

### 2.2 : Occurrence of CID during the product consumption phase

**Table 8 : Occurrence of CID during the product consumption phase - 12 weeks of study product consumption, Intent-to-Treat Population**

| | | ACTIMEL^{®} | Control | All |
|---|---|---|---|---|
| Number of subjects | | (N=500) | (N=500) | (N=1000) |
| Occurence of CID | Yes | 213 (43%) | 256 (51%) | 469 (47%) |
| | | | | |
| | No | 287 (57%) | 244 (49%) | 531 (53%) |

| | | | | |
|---|---|---|---|---|
| Logistic regression model - Least Squares Means and differences : ACTIMEL ^{®} - Control : p = 0,005 | | | | |

### EXAMPLE 3: BIOLOGICAL RESULTS

Results shown below on all biological data come from covariance analysis. More precisely the statistical analysis is a change from baseline adjusted on the the baseline value (in line with ICH recommendation). The raw change was expressed as: Biological value for CID - Biological value at baseline, calculated for each subject and statistical model is applied on available data. In addition baseline description is provided for information on the whole ITT population with CID.

### 3.1. Natural Killer cells : NK cells CD16+/56+/3- change from baseline in case of CID (parameter=NK cells CD16+/56+/3- absolute count).

A clinically relevant and statistically significant higher NK cells CD16+/56+/3- absolute count was observed in the ACTIMEL^{®} group during the product consumption phase in case of CID, for all CID (change from baseline = +45.048 cells/µl for ACTIMEL^{®} *vs*. -14.500 cells/µl for Control; p <0.001), for URTI (p= 0.016), for LRTI (p= 0.009), for rhinopharyngitis (p= 0.011), and for sore throat (p= 0.032) in ITT population (Table 9).

**Table 9: Intent-to-Treat Population restricted to the biological sub-population with CID/ test Anova**

| | | ACTIMEL^{®} | CONTROL | ALL | |
|---|---|---|---|---|---|
| Time = baseline (v2) | | | | | |
| | N | 52 | 63 | 115 | |
| NK cells CD16+/56+/3-absolute count (/µl) | Mean | 175.88 | 181.63 | 179.03 | P-value = 0.503 |
| | SD | 74.21 | 83.04 | 78.88 | |
| | | | | | |

| Changes versus baseline, Phase = Consumption Phase, All blood samples related to CID | | | | | |
|---|---|---|---|---|---|
| | | ACTIMEL^{®} | CONTROL | ALL | |
| ALL CID | N | 42 | 50 | 92 | |
| NK cells CD16+/56+/3-absolute count (/µl) | Mean | +45.048 | -14.500 | +12.685 | P-value < 0.001 |
| | SD | 80.846 | 68.999 | 79.987 | |
| | | | | | |

| Category = URTI | N | 34 | 39 | 73 | |
|---|---|---|---|---|---|
| NK cells CD16+/56+/3-absolute count (/µl) | Mean | +34.882 | -15.590 | +7.918 | P-value = 0.016 |
| | SD | 91.826 | 70.633 | 84.501 | |
| | | | | | |

| Category = LRTI | N | 13 | 17 | 30 | |
|---|---|---|---|---|---|
| NK cells CD16+/56+/3-absolute count (/µl) | Mean | +37.462 | -33.353 | -2.667 | P-value = 0.009 |
| | SD | 48.349 | 59.634 | 64.832 | |
| | | | | | |

| Type = 1 - Rhinopharyngitis | N | 23 | 27 | 50 | |
|---|---|---|---|---|---|
| NK cells CD16+/56+/3-absolute count (/µl) | Mean | +36.609 | -30.148 | +0.560 | P-value = 0.011 |
| | SD | 100.580 | 65.341 | 89.090 | |
| | | | | | |

| Type = 2 - Sore throat | N | 17 | 19 | 36 | |
|---|---|---|---|---|---|
| NK cells CD16+/56+/3-absolute count (/µl) | Mean | +23.412 | -23.368 | -1.278 | P-value = 0.032 |
| | SD | 81.050 | 73.445 | 79.612 | |

The inventors also observed (data not shown):
• A clinically relevant and statistically significant higher percentage of NK cells CD16+/56+/3- in the ACTIMEL^{®} group in case of CID, for all CID during the product consumption phase (change from baseline = +1.95 % for ACTIMEL^{®} *vs*. +0.08 % for Control; p= 0.007, for baseline values = 10.35+/-4.67 for ACTIMEL^{®} vs 9.56+/-4.29 for Control) and for LRTI during the whole study phase (p= 0.008) in ITT population.
• A statistically significant higher lytic activity of NK cells in ACTIMEL^{®} group in case of CID, for sore throat during the product consumption phase (p= 0.047 for basal activity; p= 0.013 for IL-2-stimulated activity) in ITT population.

Hence, it appears that NK cells (CD16+CD56+CD3-) absolute count is statistically different between group and higher in ACTIMEL^{®} group according to change from baseline in case of CID. This was observed, during the Product Consumption Phase for all CID, URTI and LRTI, and rhinopharyngitis and sore throat. All the results were confirmed when only the samples collected during the CID were taken into account (all with significant P).

### 3.2. Leukocytes and neutrophils change from baseline in case of CID during the product consumption phase

A clinically relevant and statistically significant higher leukocytes absolute count was observed in the ACTIMEL^{®} group [change from baseline = +1248 cells/µl for ACTIMEL^{®} vs. +165 cells/µl for Control] during the product consumption phase in case of CID, for all CID (p= 0.034) and for rhinopharyngitis (p= 0.002) in ITT population.

**Table 10 : Leukocytes (Changes from baseline) during the product consumption phase in case of CID, URTI, LRTI, rhinopharyngitis and sore throat.**

| Leukocytes (10E3/µl) | | ACTIMEL^{®} | CONTROL | ALL | |
|---|---|---|---|---|---|
| Time = baseline (v2) | N | 52 | 63 | 115 | |
| | Mean | 6.33 | 6.60 | 6.48 | P-value = 0.430 |
| | SD | 1.83 | 1.79 | 1.81 | |

| Changes versus baseline, Phase = Consumption Phase, All blood samples related to CID | | | | | |
|---|---|---|---|---|---|
| All CID | N | 42 | 51 | 93 | |
| | Mean | +1.248 | +0.165 | +0.654 | P-value = 0.034 |
| | SD | 2.630 | 2.239 | 2.470 | |

| URTI | N | 34 | 40 | 74 | |
|---|---|---|---|---|---|
| | Mean | +1.235 | +0.275 | +0.716 | P-value = 0.109 |
| | SD | 2.468 | 2.199 | 2.360 | |

| LRTI | N | 13 | 17 | 30 | |
|---|---|---|---|---|---|
| | Mean | -0.338 | +0.006 | -0.143 | P-value = 0.319 |
| | SD | 2.000 | 2.858 | 2.489 | |

| Type = 1 - rhinopharyngitis | N | 23 | 28 | 51 | |
|---|---|---|---|---|---|
| | Mean | +1.287 | -0.354 | +0.386 | P-value = 0.002 |
| | SD | 2.451 | 1.608 | 2.172 | |
| Type = 2 - sore throat | N | 17 | 20 | 37 | |
| | Mean | +0.624 | +0.500 | +0.557 | P-value = 0.682 |
| | SD | 2.544 | 2.485 | 2.478 | |

The inventors also observed a clinically relevant and statistically significant higher neutrophils absolute count *vs*. baseline in the ACTIMEL^{®} group [change from baseline = +1050 cells/µl for ACTIMEL^{®} vs. +210 cells/µl for Control] in case of CID, for all CID during the whole study phase (p= 0.037) and for rhinopharyngitis (p= 0.002) during the product consumption phase in ITT population. In case of rhinopharyngitis, the mean change value was +1279 cells/µl in ACTIMEL^{®} group and -231 cells/µl in control group for baseline values of 3740 and 3800, respectively (Table 11).

A clinically relevant and statistically significant higher neutrophils percentage was observed in the ACTIMEL^{®} group [change from baseline = +5.3% for ACTIMEL^{®} vs +1.5% for Control] in case of CID, for all CID during the whole study phase (p= 0.038) and for rhinopharyngitis (p= 0.017) during the product consumption phase in ITT population (Table 12). The variation of leucocytes count previously described is thus due to neutrophils count modification since no other cell subset in hemogram analysis was significantly modified.

**Table 11 : Neutrophils (absolute count) during the product consumption phase in case of CID; Intent-to-Treat Population restricted to the biological sub-population - with CID/ test Anova.**

| Neutrophils absolute count (10E3/µl) | | ACTIMEL^{®} | CONTROL | ALL | |
|---|---|---|---|---|---|
| Time = baseline (v2) | | | | | |
| | N | 52 | 63 | 115 | |
| | Mean | 3.74 | 3.80 | 3.77 | P-value = 0.829 |
| | SD | 1.45 | 1.26 | 1.35 | |

| Changes versus baseline, All blood samples related to CID Phase = Product consumption phase | | | | | |
|---|---|---|---|---|---|
| Type = 1 - rhinopharyngitis | N | 23 | 28 | 51 | |
| | Mean | +1.279 | -0.231 | +0.450 | P-value = 0.002 |
| | SD | 2.339 | 1.433 | 2.023 | |

**Table 12 : Neutrophils (percentage) during the product consumption phase in case of CID; Intent-to-Treat Population restricted to the biological sub-population - with CID/ test Anova.**

| Neutrophils (%) | | ACTIMEL^{®} | CONTROL | ALL | |
|---|---|---|---|---|---|
| Time = baseline (v2) | | | | | |
| | N | 52 | 63 | 115 | |
| | Mean | 57.85 | 57.19 | 57.49 | P-value = 0.591 |
| | SD | 7.78 | 8.36 | 8.08 | |

| Changes versus baseline, All blood samples related to CID Phase = Product consumption phase | | | | | |
|---|---|---|---|---|---|
| Type = 1 - rhinopharyngitis | N | 23 | 28 | 51 | |
| | Mean | +6.609 | -0.071 | +2.941 | P-value = 0.017 |
| | SD | 10.659 | 8.641 | 10.079 | |

### CONCLUSION

In a sub-population highly sensitive to respiratory infections such as smokers, ACTIMEL^{®} reduced the number of all CIDs. In the global study population, a statistically significant difference (Table 8) was also observed in favour of ACTIMEL^{®} in the occurrence of CID during the product consumption phase of the study (both in the ITT and the PP population).

In case of CID, ACTIMEL^{®} consumption also increased the time to the first occurrence of infection and reduces the cumulated duration of associated fever (only during whole study phase for fever).

These clinical effects are associated with an effect of ACTIMEL^{®} on several immune cells subset since ACTIMEL^{®} increased significantly the number of blood leukocytes, neutrophils and NK cells.

The effect of ACTIMEL^{®} on immune parameters is consistent with its observed capacity to improve clinical outcomes in case of CID and this may constitute an interesting base of results to further investigate the mechanisms of action of ACTIMEL^{®}

### REFERENCES

Agarwal K.N., Bhasin S.K., Faridi M.M., Mathur M. and Gupta S. 2001. Lactobacillus casei in the control of acute diarrhea-a pilot study. Indian Pediatrics; 38, 905-910
Curti R, Radice L, Cesana GC, Zanettini R, Grieco A. 1982. Work stress and immune system: lymphocyte reactions during rotating shift work. Preliminary results. Med Lav. 73:564-569
Feeney A, North F, Head J, Canner R, Marmot M. 1998. Socioeconomic and sex differentials in reason for sickness absence from the Whitehall II study. Occup Environ Med.55:91-98
Greenberg SB. 2002. Respiratory viral infections in adults. Curr opin Pulm Med. 8:201-208
Kobayashi F, Furui H, Akamatsu Y, Watanabe T, Horibe H. 1997. Changes in psychophysiological functions during night shift in nurses. Influence of changing from a full-day to a half-day work shift before night duty. Int Arch Occup Environ Health. 69:83-90
Marcos A., Wärnberg J., Nova E., Gomez S., Alvarez A., Alvarez R., Mateos J.A., and Cobo J.M. 2004. The effect of milk fermented by yogurt cultures plus Lactobacillus casei DN-114001 on the immune response of subjects under academic examination stress. Eur. J. Nutr. 43[6]:381-389
Mohren DC, Jansen NW, Kant IJ, Galama JM, van der Brandt PA, Swaen GM. 2002. Prevalence of common infections among employees in different work schedules. J Occup Environ Med. 44:1003-1011
Parra D, De-Morentin-B-Martinez, Cobo-J-M, Mateos-A, Martinez-J-A. 2004 a. Monocyte function in healthy middle-aged people receiving fermented milk containing Lactobacillus casei. J-Nutr-Health-Aging, 8, 4, 208-11.
Parra D, Martinez-de-Morentin-B-E, Cobo-J-M, Mateos-A, Martinez-J-A. 2004 b. Daily ingestion of fermented milk containing Lactobacillus casei DN 114001 improves innate-defense capacity in healthy middle-aged people. J-Physiol-Biochem, 60, 2, 85-91.
Pedone C.A., Arnaud C.C., Postaire E.R., Bouley C.F. and Reinert P. 2000. Multicentric study of the effect of milk fermented by Lactobacillus casei on the incidence of diarrhoea. Int. J. Clin. Pract. 54, 568-571
Pedone C.A., Bemabeu A.O., Postaire E.R., Bouley C.F. and Reinert P. 1999. The effect of supplementation with milk fermented by Lactobacillus casei (strain DN-114 001) on acute diarrhoea in children attending day care centres. Int. J. Clin. Pract. 53, 179-184
Pujol, P., Huguet, J., Drobnic, F., Banquells, M., Ruiz, O., Galilea, P., Segarra, N., Aguilera, S., Bumat, A., Mateos, J. A. and Postaire, E. R. 2000. The effect of fermented milk containing Lactobacillus casei on the immune response to exercise. Sports Med., Training and Rehab. 9[3], 209-223
Tubelius P, Stan V, Zachrisson A. 2005. Increasing work-place healthiness with the probiotic Lactobacillus reuteri: a randomised, double-blind control-controlled study. Environ-Health vol. 4, p. 25
Turchet P., Laurenzano M., Auboiron S., Antoine J.M. 2003. Effect of fermented milk containing the probiotic Lactobacillus casei DN-114 001 on winter infections in free-living elderly subjects: a randomised, controlled pilot study. J. of Nutr., Health & Aging; 7[2], 75-77

## Claims

1. Use of a *Lactobacillus casei* strain, for the preparation of a composition for improving the resistance of a tobacco user against a common infectious disease, especially against a respiratory common infectious disease.

2. The use of claim 1, wherein said tobacco user is a smoker.

3. The use of claim 1 or claim 2, wherein said tobacco user is younger than 65 years old.

4. Use of a *Lactobacillus casei* strain, for the preparation of a composition for improving the cellular immune response of an individual in case of respiratory common infectious disease.

5. The use of claim 4, wherein said individual is younger than 65 years old.

6. The use of claim 4 or claim 5, for the preparation of a composition for improving the NK cells response in case of respiratory common infectious disease.

7. The use of any of claims 4 to 6, for the preparation of a composition for improving the neutrophilic leukocytes in case of respiratory common infectious disease.

8. The use of any of claims 4 to 7, wherein said respiratory common infectious disease is rhinopharyngitis.

9. The use of any of claims 4 to 7, wherein said respiratory common infectious disease is sore throat.

10. The use of any of claims 1 to 9, wherein said *Lactobacillus casei* strain is a *Lactobacillus casei* ssp. *paracasei* strain.

11. The use of claim 10, wherein said *Lactobacillus paracasei* strain is the strain which was deposited at the CNCM under the reference 1-1518.

12. The use according to any of claims 1 to 11, wherein said composition comprises at least 1 × 10⁵ c.f.u. per millilitre of said *Lactobacillus casei* strain.

13. The use according to any of claims 1 to 11, wherein said composition comprises at least 1 × 10⁷ c.f.u. per millilitre of said *Lactobacillus casei* strain.

14. The use according to any of claims 1 to 13, wherein the composition further contains at least one bacteria selected from the genera *Lactobacillus, Lactococcus, Streptococcus and Bifidobacterium.*

15. The use according to claim 14, wherein said composition comprises *Lactobacillus bulgaricus* and/or *Streptococcus thermophilus* bacteria.

16. The use according to any of claims 1 to 15, wherein said composition is in the form of an aliment.

17. The use according to any of claims 1 to 16, wherein said composition is a fermented milk composition.
